# EUROPEAN PATENT SPECIFICATION

(11) **EP 1 486 271 B1**
(45) Date of publication and mention of the grant of the patent: **16.07.2008**
(21) Application number: 03013291.4
(22) Date of filing: 13.06.2003
(51) Int. Cl.: B22D 2/00, B22D 11/18, B22D 37/00, G01N 27/74, G01N 33/20, C21C 5/46

(54) **A method and a device for detecting slag**
Verfahren und Vorrichtung zum Erkennen von Schlacken
Procédé et dispositif de détection de laitier

(43) Date of publication of application: 15.12.2004
(73) Proprietor: MPC Metal Process Control AB, 611 26 Nyköping (SE)
(72) Inventor: Jalk, Mats, 611 26 Nyköping (SE); Ohlsson, Willy, 611 31 Nyköping (SE); Kelvesjö, Hakan, 611 26 Nyköping (SE)
(74) Representative: Lind, Urban Arvid Oskar

(56) References cited:
- WO-A-02/36293
- DE-A- 3 142 681
- JP-A- 54 110 932
- JP-A- 54 119 336
- US-A- 4 144 756
- US-A- 4 816 758
- PATENT ABSTRACTS OF JAPAN vol. 1998, no. 05, 30 April 1998 (1998-04-30) & JP 10 005958 A (NIPPON STEEL CORP), 13 January 1998 (1998-01-13)
- PATENT ABSTRACTS OF JAPAN vol. 1998, no. 01, 30 January 1998 (1998-01-30) & JP 09 236461 A (NIPPON STEEL CORP), 9 September 1997 (1997-09-09)

## Description

### Technical field of the Invention

The present invention relates to a method and a device for detecting slag in a flow of molten metal. In particular, the invention relates to detecting the presence of slag by means of an electromagnetic field which is propagated through the molten metal. The invention also relates to a casting plant comprising such a device.

### Background of the Invention

In the metallurgical industry there are different processes in which liquid metal is to be processed in one way or another. One example is the casting of metal, such as steel. In part of such a casting process, the liquid metal is supplied from a ladle to a tundish via a pouring nozzle which is generally referred to as a shroud. The metal flows from the tundish via another pouring nozzle to a casting mould or chill mould, in which the metal is cooled and transformed into solid form.

In smelting and heating processes there is a risk of slag being formed in the molten metal. In order to minimize the risk of slag being included in the final cast product, the casting plant may include a slag detection device. If the slag detection device detects slag in the molten metal an alarm is generated so that suitable measures may be taken.

In the prior art one type of slag detection device includes electromagnetic coils placed above a sliding gate in the ladle. This type of slag detection device works on the principle that for an electromagnetic field the penetration depth of metal is much less than the penetration depth of slag. Thus, a large portion of the electromagnetic field is allowed to pass through slag, similarly to air.

Even though the prior art detecting device generally provides satisfactory detecting results, it has some drawbacks. One drawback is that every ladle used in a casting plant has to be equipped with the electromagnetic coils, the coils being welded to the ladle. Furthermore, the coils installed in a ladle are in such a position where there is a relatively high risk of mechanically damaging the sensors. Thus, it would be desirable to install electromagnetic slag detecting sensors (coils) in such manner that cut costs and minimizes the risk of mechanical damage to the sensors.

In JP 54 110932 A a slag detector for a molten steel passage is shown. The device comprises a forked coil holder having two branches. The electromagnetic coils may then be held by respective branches adapted to be placed on respective sides of the passage for molten steel. The forked coil holder is connected to a separate manipulator. A drawback with this known device is that more accurate detecting results are desirable.

### Summary of the Invention

An object of the present invention is to achieve a method, a device and a casting plant which alleviate the drawbacks of the prior art.

This and other objects, which will become apparent in the following, are accomplished by a method, a device and a casting plant as defined in the accompanied claims.

The invention is based on the insight that by optimising the constancy of the propagation of an electromagnetic field through a flow of molten metal, it is possible to move the electromagnetic slag detection out of the ladle. In particular, by controlling the molten metal to be subjected to a positionally constant electromagnetic field, it is even possible to perform the slag detection at such location which moves as the molten metal passes said location. In fact, it has been found that a shroud which guides molten metal from the ladle to a tundish can be subjected to electromagnetic slag detection, in a simple and economical manner, despite the movements of the shroud. Movement of the shroud may occur during the pouring of the molten metal. Such movement may be linear or in arcs of a circle. The shroud also tends to move when the sliding gate at the ladle is moved.

Consequently, according to one aspect of the invention, there is provided a method of detecting the presence of slag in a channel for guiding molten metal. According to this method a transmitting coil is used for generating an electromagnetic field which enters the channel and its contents. A receiving coil is used to receive the electromagnetic field that has entered the channel and its contents. The electromagnetic field generates an induced voltage in the receiving coil. The value of the induced voltage, e.g. if it is outside or within a defined voltage range, is indicative of the presence or absence of slag in said contents. The electromagnetic field generated by the transmitting coil(s) is arranged to enter a shroud and its contents, the shroud being the above-mentioned channel for guiding molten metal, in particular from a ladle to a tundish. The transmitting and receiving coils are kept substantially unmovable relative to the shroud. Thus, the method enables detection of the presence of slag in the shroud.

According to a second aspect of the invention, there is provided a device for detecting slag in line with the above mentioned method.

The transmitting coil and the receiving coil are suitably held in place by a common or separate coil holders. The common coil holder is designed in the form of a fork having at least two branches. The transmitting coil and the receiving coil may then be held by respective branches adapted to be placed on respective sides of the shroud. The forked coil holder is connected to other equipments as will be explained below.

During the process of slag detection the coils are kept unmovable relative to the shroud. If the coils are unmovable relative to the shroud, i.e. they follow any movement of the shroud, an even signal is obtained for an unchanged flow of molten metal. This may be accomplished by e.g. mounting the transmitting coil and the receiving coil to respective branches of a forked coil holder mentioned above. In that case, the forked coil holder is suitably placed in such manner that an imagined straight line drawn between the transmitting coil and the receiving coil crosses the shroud. In other words the shroud is placed between the branches of the forked coil holder. An advantage of using a forked coil holder is that it is possible to use a relatively long stem thereof, wherein positioning of the coils around the shroud can be accomplished by manoeuvring at a distance from the shroud without having to access the shroud closely. The branches of the forked coil holder facilitate correct positioning of the coils and reduces the risk of relative movements between the coils thereby avoiding unnecessary erroneous signal variations. The risk of relative movements between the coils are further reduced if the branches are made relatively short. Thus, a relatively long stem having short branches may suitably be used. Since the forked coil holder, preferably, does not entirely surround the circumference of the shroud it is easily locatable to and removable from the shroud.

In order to enable the coils to substantially follow any movement of the shroud, the forked coil holder is suitably suspended to an arrangement that follows the movements of the shroud. One example of such an arrangement is a shroud manipulator of any known type. A shroud manipulator is generally in the form of an elongate arm that is used to fetch, position and support the shroud. The forked coil holder may thus be mounted to such a shroud manipulator. As an alternative, the forked coil holder may be suspended to the sliding gate unit mounted on the ladle, and other alternatives are also possible in order to achieve the desired motion following effect.

The two branches that hold the transmitting coil and the receiving coil respectively may in at least one embodiment of the invention be electrically isolated from one another. If for example the branches are connected to the stem by means of a common cross bar, that bar may be provided with electrical isolation. This type of interruption minimizes unwanted electromagnetic fields.

According to at least one embodiment of the invention, as an alternative to providing transmitting and receiving coils on different sides of the shroud, each coil is arranged to surround the shroud. The coils may be given a toroid or annular form and the shroud will extend through each toroid. The coils may be mounted between the branches of a forked coil holder which in turn is mounted to a shroud manipulator or other shroud following arrangement.

It should be clear from the above discussion that regardless of the type of coil arrangement chosen, it is possible to arrange the coils in such manner that the coils are enabled to substantially follow any positional variations of the shroud. As previously explained, the advantage of this is that the propagation of the generated magnetic field entering the shroud will be constant, wherein the accuracy of the detection is increased even though the shroud moves during the pouring of the molten metal.

The voltage induced by the receiving coil(s) is used to determine whether or not slag or slag is present. According to at least one embodiment of the invention, any induced voltage having a value outside a defined voltage range is indicative of the presence of slag. The flow of molten metal passing through the shroud is determined and the voltage range is defined depending on the magnitude of the determined flow of molten metal. The advantage of this is that the detection of slag in the shroud may be even more accurate. A shroud is not necessarily completely filled with molten metal at all times. Since gas and slag have similar influence or lack of influence on the electromagnetic field, some gas in the shroud could be taken for slag. According to said embodiment an alarm limit is set depending on the amount of gas (e.g. air) present. In particular, the embodiment is based on the insight that the presence of gas can be determined by determining the flow, i.e. the volume per unit of time, of molten metal passing through the shroud.

The advantage of determining the flow of molten metal is that it provides a ratio of the flow components, i.e. the amount of metal to the amount of gas in the shroud, wherein depending on a change in said ratio the sensitivity of the detection device may be varied. For instance, if the flow of molten metal decreases after a while, the requirement for or level of providing an alarm of the detection device is increased. This is done by increasing said defined voltage range. This means that a given induced voltage may be either indicative of the presence of slag or not depending on the presently defined voltage range. Thus, on the one hand a given induced voltage value may fall outside a voltage range defined before a decrease of the flow, which would result in an indication of presence of slag and on the other hand, after the decrease of the flow and the increase of the voltage range, the same value of induced voltage would be covered by the redefined voltage range, wherein it would be construed as presence of gas, if any, and not slag. The risk of slag being entrained in the flow of molten metal usually increases towards the end of the process, when the ladle is close to being drained.

The variation of voltage range may be illustrated in the following way. Suppose that the receiving coil, for an empty shroud, i.e. just gas and no molten metal, generates an induced voltage having a signal strength V₁. When molten metal passing through the shroud completely fills up the shroud a voltage having a signal strength V₂ will be induced, wherein V₂ < V₁. Thus, for a filled shroud the voltage range is set to at least the interval 0-V₂, however, in order to be on the safe side and not risk a false alarm, the voltage range may be extended somewhat. When the shroud is only partly filled with molten metal, the definition of the voltage range is suitably adjusted. The newly defined voltage range will be 0-V₃, wherein V₂ < V₃ < V₁.

A simple calculation example will now follow. Suppose that for a shroud completely filled with molten metal the voltage range is defined such that the slag is only indicated as being present if the magnitude of the induced voltage indicates a metal content of 90% or less (while the determined magnitude of flow of molten metal is still about 100%). Thus, if the induced voltage corresponds to a shroud filled by 95% with molten metal, there will be no slag indication. This provides a 10% safety margin for avoiding false alarm. Now, suppose that later on it is determined that the flow of molten metal has decreased to half, i.e. only 50% of the shroud is filled with molten metal and the rest is filled with gas. In order to maintain the 10% safety margin the voltage range is defined such that the presence of slag is indicated if the value of the induced voltage corresponds to a metal content of 45% or less. Note that the numbers in the this example are merely exemplifying in order to illustrate a general principle of varying the voltage range.

As previously mentioned, the flow of molten metal guided through the shroud may be determined either directly or indirectly. Different types of flow meters may be employed for direct measurement of the flow inside the shroud. However, it may be simpler to make use of an indirect method of determination. For instance, the flow of molten metal passing through the shroud may be determined by measuring the teeming rate in the tundish and calculating the flow of molten metal from said measured teeming rate. Another alternative is to measure the rate of decrease in weight of the ladle content and calculating the flow of molten metal from said measured rate of decrease in weight. Yet another alternative is to measure the casting speed and the dimension of the cast product after the chill mould, the speed and dimension being indicative of the flow. A further alternative is to detect the degree of opening or the position of the sliding gate mounted under the ladle and to provide a feedback signal to a processor, which calculates the flow of molten metal from the positional information of the sliding gate, i.e. how much the siding gate has been opened. An advantage of these different alternatives is that standard existing measuring systems may be used to provide the relevant information desired to define slag alarm settings.

In at least one embodiment of the invention, as a supplement to the control of the defined voltage range, the frequency of the electromagnetic field generated by the transmitting coil may be controlled. It has been found that a higher frequency provides a more stable induced voltage signal when the shroud is not completely filled and a turbulent flow of molten metal is present inside the shroud. Thus, when a turbulent flow inside the shroud has been detected, the frequency of the electromagnetic field is changed, suitably increased, by the system. Also, the magnitude of the flow of molten metal influences the chosen frequency in order to penetrate the metal and detect any slag in the centre of or at any other place if the flow of molten metal. External frequencies that can interfere with the measurement can be a ground for changing the frequency. Thus, the electromagnetic field generated by the transmitting coils(s) may be of alternating frequencies. Also, several coils may be used for generating several electromagnetic fields, each with a different frequency, in order to minimise noise effects. Furthermore, several coils may be used for generating several electromagnetic fields, one or more being of alternating frequencies.

The transmitting coil that generates the electromagnetic field may be provided with directional elements, such as a core, for optimised direction of the electromagnetic field towards the portion of the shroud in which it is intended to check whether or not slag is present. Similarly the receiving coil may be provided with such directional elements.

Due to the high temperature environment, according to at least one embodiment of the invention, the transmitting and receiving coils may be cooled by means of a suitable cooling arrangement, such as cooling channels containing water or gas.

According to the invention, there is provided a device for detecting the presence of slag in a shroud which guides molten metal from a ladle to a tundish, the device comprising a forked coil holder having at least two branches, a first branch carrying a transmitting coil and a second branch carrying a receiving coil, the two branches being placeable in such manner that the shroud is located between them. This enables the coils to be substantially unmovable relative to each other and the shroud and the contained metal stream, thereby avoiding unwanted signal variations Another advantage is that the coils are independent of the ladle.

According to another aspect of the invention, there is provided a casting plant, which comprises a ladle, a tundish and a shroud between them. The casting plant also comprises a device for detecting the presence of slag inside the shroud, as has been previously described in this application.

### Brief description of the drawings

The invention will now be described with reference to the accompanied drawings, in which:
Fig. 1 is a schematic illustration of a device arranged to detect slag in a shroud in accordance with at least one embodiment of the invention.
Fig. 2 is a more detailed schematic illustration of the components shown around the shroud in Fig. 1.
Fig. 3 is a flow chart illustrating an example of an operation of a control unit.

### Detailed description of the drawings

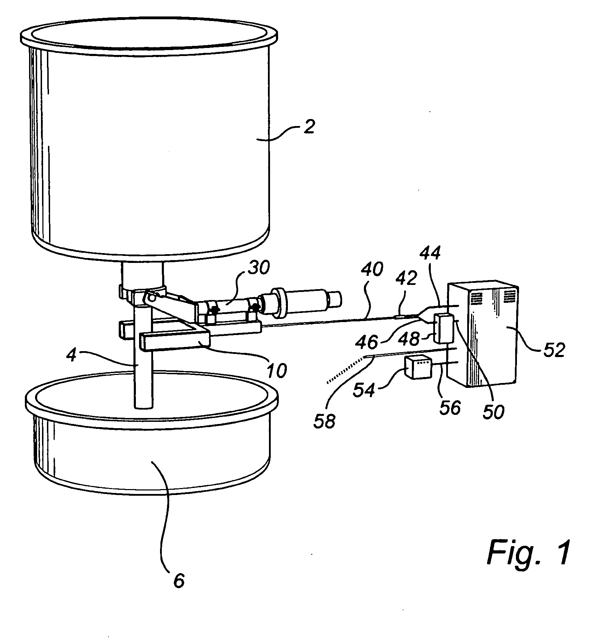

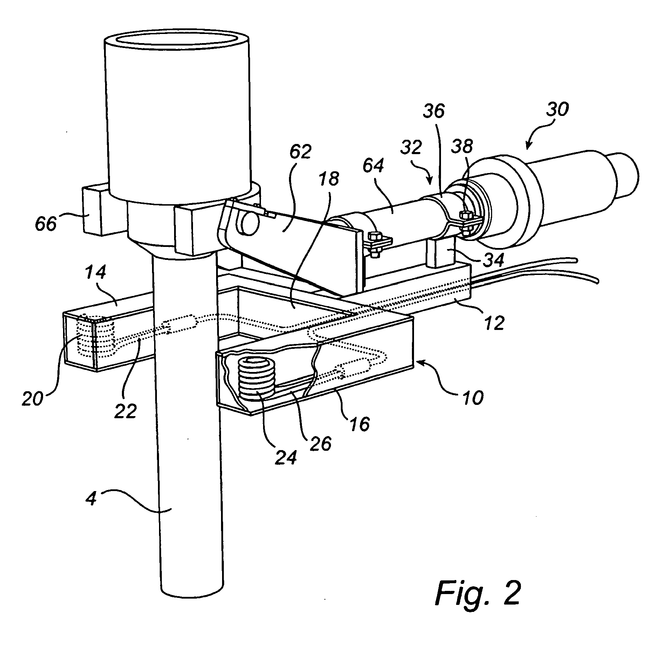

Fig. 1 schematically illustrates a ladle 2 adapted to contain molten metal which is to be passed through a shroud 4 over to a tundish 6. The metal flows from the tundish 6 via a pouring nozzle to a chill mould, in which the metal is cooled and transformed into solid form, the pouring nozzle and the chill mould not being shown in the figure. A forked coil holder 10 is provided at the outside of the shroud 4 and is mounted to a shroud manipulator 30. The forked coil holder 10 contains a transmitting coil for generating an electromagnetic field to be propagated through the shroud and to be received by a receiving coil which is also located in the forked coil holder 10. The forked coil holder 10 and the coils will be further described in connection with the discussion of Fig. 2.

Continuing with Fig. 1, a signal cable 40 extends between the forked coil holder 10 and a connector 42. The signal cable 40 contains internal cables or separate wires for leading signals to and from the transmitting coil and the receiving coil, respectively. Also connected to the connector 42 are a transmitter signal cable 44 and a receiver signal cable 46 containing wires for providing signals to and from the transmitting coil and the receiving coil, respectively. The receiver signal cable 46 is at its other end connected to a preamplifier unit 48 in order to amplify generated induced voltage signals from the receiving coil. A preamplifier cable 50 passes on the signals to a control unit 52 which comprises a processor. The transmitter signal cable 44 is directly connected to the control unit 52. The generation of an electromagnetic field by the transmitting coil can thus be controlled by sending a signal from the control unit 52, via the transmitter signal cable 44, the connector 42 and the associated wires in the signal cable 40 to the transmitting coil inside the forked coil holder 10.

The control unit 52 is connected to an alarm unit 54 via an alarm cable 56. The control unit 52 compares the received induced voltage signal from the receiving coil with a predefined voltage range. If the received voltage is not included in said voltage range, the control unit 52 activates the alarm unit 54, which may suitably generate an acoustic and/or visual alarm. Also, even though not shown, a control panel may be provided for enabling continuous visual monitoring of the slag detection process.

Furthermore, the control unit 52 receives information about the flow, i.e. the volume per unit of time of molten metal through the shroud. This piece of information is received over a flow input line 58 which at its other end is connected to a flow determining device (not shown), which can be either a direct measuring device or an indirect measuring device with subsequent calculation, as previously explained. If it is determined that a significant change in flow of molten metal has occurred, the control unit 52 will set a different voltage range. A decreased flow results in the setting of a larger voltage range and an increased flow results in the setting of a narrower voltage range. Thus, the point at which the alarm unit 54 is activated is dependent on the received flow information.

In Fig. 2 the forked coil holder 10 and its suspension to the shroud manipulator 30 holding the shroud 4 is shown in more detail. Fig. 2 is a partly cut away perspective view. The forked coil holder 10 comprises a stem portion 12, a first branch portion 14, a second branch portion 16 and a cross bar portion 18. In this embodiment the branch portions 14, 16 are located at opposite ends of the cross bar portion 18 and the stem portion 12 is connected to the middle of the cross bar portion 18. However, in alternative embodiments the stem portion could be placed at one end of the cross bar portion and even be an elongation of one of the branch portions.

Fig. 2 illustrates that the interior of the first branch portion 14 comprises at its end farthest from the cross bar portion 18 a transmitting coil 20. The wires 22 of the transmitting coil are passed through the first branch portion 14, via the cross bar portion 18 and the base portion 12 to the control unit (see also Fig. 1).

Fig. 2 also illustrates that the interior of the second branch portion 16 comprises at its end farthest from the cross bar portion 18 a receiving coil 24. The wires 26 of the receiving coil 24 are passed through the second branch portion 16, via the cross bar portion 18 and the base portion 12 to the control unit (see also Fig. 1).

The ends of the branches portions 14, 16 are so positioned that the transmitting coil 20, the shroud 4 and the receiving coil 24 are located in a straight line. In other words, the shroud 4 is located between the two coils 20, 24. The branch portions 14, 16, or sections thereof, may be controllable between different angles relative to the rest of the forked coil holder. This enables the coils 20, 24 to be correctly positioned for setting the main direction of propagation of the electromagnetic field at a right angle to the shroud in working position.

The wires of the two coils 20, 24 may be electrically isolated from one another in the cross bar portion 18 and the stem portion 12 in order to avoid unwanted electromagnetic influence on any signals.

The forked coil holder 10 is suitably made of mainly nonmagnetic material, such as any one of stainless steel, ceramic material, fibre glass or other similar suitable material that substantially does not interfere with the electromagnetic field.

The forked coil holder 10 is suspended to the shroud manipulator 30 by means of a clamping arrangement 32 comprising two protrusions 34 each of them having at its end a clamp 36 surrounding the shroud manipulator 30. The clamps 36 are tightened to the shroud manipulator 30 by means of bolts or screws 38.

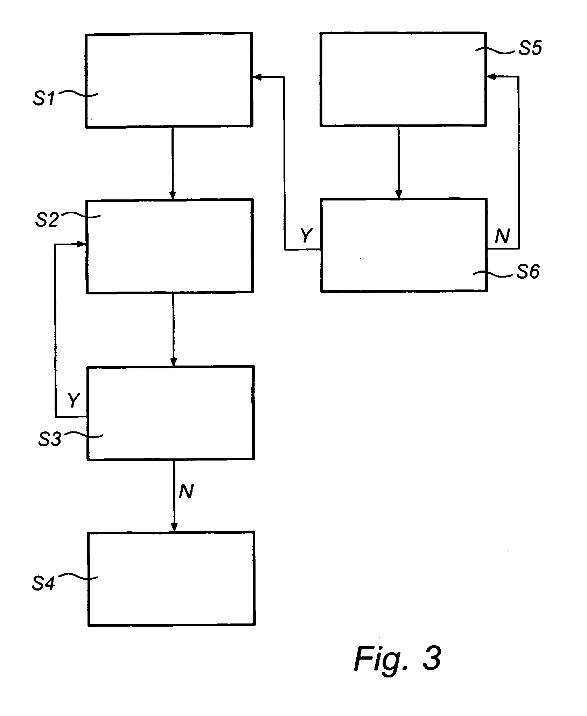

The shroud manipulator 30 illustrated in Fig. 2 is of standard type and comprises at the end portion that holds the shroud 4 a gripping means. The gripping means comprises two grip portions 62 that are inclined upwards from the rear elongate portion 64 of the shroud manipulator 30. The inclination depends among other things on how the shroud is to be positioned. The initial gripping point on the shroud differ from the shroud positioning point of the manipulator, making it desirable to change the angle. The grip portions 62 are further connected to a holder 66 shaped as a horse shoe. Fig. 3 is a flow chart illustrating an example of an operation of the control unit 52 shown in Fig. 1.

In a step S1 the control unit defines a voltage range. In a step S2 the control unit receives a signal indicative of the voltage induced at the receiving coil. In a step S3 the control unit compares the value of the received signal with the defined voltage range and determines whether or not said value is within the defined voltage range. If said value is not within the defined voltage range (in such a case the absolute value of the received signal is generally larger then the absolute value of the maximum voltage of the voltage range), then the control unit activates a slag detection alarm in a step S4. If on the other hand it is determined in step S3 that the value of the received signal is encompassed by the defined voltage range, the control unit returns to step S2 to receive a new signal to be compared in the subsequent step S3 etc.

In parallel to said steps, the control unit also receives a flow signal in a step S5. The flow signal is indicative of the volume per unit of time of molten metal flowing through the shroud. In a step S6 the control unit compares the determined flow of molten metal with stored values and examines whether or not there has been a significant change of flow over time. If there has been no significant change the control unit continues with the step S5 to examine subsequent flow signals. However, if there is a significant change, the control unit will in accordance with the stored values (such as a table) redefine the voltage range in the step S1.

It should be noted that Fig. 3 is only an example of the operation of the control unit for elucidating purposes and that other alternatives are also possible.

It should also be noted that numerous modifications and variations can be made without departing from the scope of the present invention defined in the accompanied claims. For instance, even though the description is focused on the use of one transmitting coil and one receiving coil, the method and device according to the invention may comprise several transmitting coils and several receiving coils.

## Claims

1. A method of detecting the presence of slag in a shroud (4) for guiding molten metal from a ladle (2) to a tundish (6), comprising:
providing a forked coil holder (10) having at least two branches;
mounting said at least one transmitting coil (20) to a first branch (14) and said at least one receiving coil (24) to a second branch (16) of the forked coil holder;
placing the forked coil holder in such manner that an imagined straight line drawn between said at least one transmitting coil and said at least one receiving coil crosses the shroud;
generating, by means of at least one transmitting coil (20), an electromagnetic field that enters the shroud and its contents;
generating an induced voltage by means of at least one receiving coil (24) which is subjected to the electromagnetic field having entered the shroud and its contents, wherein said induced voltage is indicative of the presence or absence of slag in said contents;
**characterized by**
mounting said forked coil holder (10) to a shroud manipulator (30) adapted to manipulate the shroud; and thereby
keeping the coils (20, 24) unmovable relative to the shroud.

2. The method as claimed in claim 1, further comprising:
providing said at least one transmitting coil in toroid form and arranging it so as to surround the shroud, and
providing said at least one receiving coil in toroid form and arranging it so as to surround the shroud.

3. The method as claimed in any one of claims 1-2, further comprising:
detecting turbulent flow, if any, inside the shroud; and
changing the frequency of the electromagnetic field generated by said at least one transmitting coil in case of turbulent flow having been detected.

4. The method as claimed in any one of claims 1-3, further comprising generating, by means of said at least one transmitting coil:
an electromagnetic field of alternating frequencies, or
several electromagnetic fields with different frequencies.

5. The method as claimed in any one of claims 1-4, wherein any induced voltage having a value outside a defined voltage range is indicative of the presence of slag, the method further comprising:
determining the flow of the molten metal passing through the shroud; and
defining said voltage range depending on the magnitude of the determined flow of molten metal.

6. The method as claimed in claim 5, further comprising:
defining a larger voltage range if it is determined that the magnitude of the flow of molten metal has decreased.

7. The method as claimed in any one of claims 5 - 6, wherein the act of determining the flow of molten metal passing through the shroud comprises:
providing feedback from an opening position signal of a sliding gate at the ladle and calculating the flow of molten metal from the sliding gate opening information.

8. The method as claimed in any one of claims 5 - 6, wherein the act of determining the flow of molten metal passing through the shroud comprises:
measuring the rate of decrease in weight of the ladle content and calculating the flow of molten metal from said measured rate of decrease in weight.

9. The method as claimed in any one of claims 5 - 6, wherein the act of determining the flow of molten metal passing through the shroud comprises:
measuring the teeming rate in the tundish and calculating the flow of molten metal from said measured teeming rate.

10. The method as claimed in any one of claims 1 - 9, further comprising:
cooling said transmitting and receiving coils.

11. A device for detecting the presence of slag in a shroud (4) for guiding molten metal from a ladle (2) to a tundish (6), comprising:
at least one transmitting coil (20) for generating an electromagnetic field to be entered into the shroud and its contents;
at least one receiving coil (24) for receiving the electromagnetic field that has entered the channel and its contents, and for generating an induced voltage, wherein said induced voltage is indicative of the presence or absence of slag in said contents;
a shroud manipulator (30) adapted to manipulate the shroud;
a forked coil holder (10) having at least two branches, a first branch (14) carrying said at least one transmitting coil and a second branch (16) carrying said at least one receiving coil, the two branches being placeable in such manner that the shroud is located between them; **characterized in that** the forked coil holder (10) is mounted to the shroud manipulator (30) such that said at least one transmitting coil and said at least one receiving coil are in a stationary position in relation to the shroud.

12. The device as claimed in claim 11, wherein said coils are in the form of toroids, wherein the forked coil holder (10) is adapted to hold each toroid in such manner that it surrounds the shroud.

13. The device as claimed in any one of claims 11 - 12, wherein said two branches are electrically isolated from each other.

14. The device as claimed in any one of claims 11-13, wherein any induced voltage having a value outside a defined voltage range is indicative of the presence of slag, the device further comprising:
means for determining the flow of the molten metal passing through the shroud; and
means (52) for defining said voltage range depending on the magnitude of the measured flow.

15. The device as claimed in claim 14, wherein said means for determining the flow of the molten metal passing through the shroud comprises:
a sensor for sensing an opening position signal of a sliding gate at the ladle, and
a processor for calculating the flow of molten metal from the sliding gate opening information.

16. The device as claimed in claim 14, wherein said means for determining the flow of the molten metal passing through the shroud comprises:
a measuring device for measuring the rate of decrease in weight of the ladle content, and
a processor for calculating the flow of molten metal from said measured rate of decrease in weight.

17. The device as claimed in claim 14, wherein said means for determining the flow of the molten metal passing through the shroud comprises:
a measuring device for measuring the teeming rate in the tundish, and
a processor for calculating the flow of molten metal from said measured teeming rate.

18. The device as claimed in any one of claims 11 - 17, wherein the transmitting and receiving coils are provided with directional elements, such as a core, for directing the electromagnetic field towards and from the shroud.

19. A casting plant, comprising
a ladle (2) adapted to contain molten metal;
a tundish (6) adapted to receive molten metal from the ladle;
a shroud (4) arranged between the ladle and the tundish, wherein molten metal is enabled to pass from the ladle, through the shroud, and to the tundish; and
a device as claimed in any one of claims 11 - 18.

## Patentansprüche

1. Verfahren zum Erkennen des Vorhandenseins von Schlacke in einem Rohr (4) zum Führen von schmelzflüssigem Metall von einer Pfanne (2) zu einer Gießwanne (6), mit den Schritten:
Bereitstellen eines Gabelspulenhalters (10) mit mindestens zwei Zweigen;
Befestigen mindestens einer Sendespule (20) an einem ersten Zweig (14) und mindestens einer Empfangsspule (24) an einem zweiten Zweig (16) des Gabelspulenhalters;
Anordnen des Gabelspulenhalters so, dass eine imaginäre Gerade, die zwischen der mindestens einen Sendespule und der mindestens einen Empfangsspule gezogen wird, das Rohr schneidet;
Erzeugen, mit der mindestens einen Sendespule (20), eines elektromagnetischen Felds, das an das Rohr und seinen Inhalt angelegt wird;
Erzeugen einer induzierten Spannung mittels der mindestens einen Empfangsspule (24), die dem elektromagnetischen Feld ausgesetzt wird, das an das Rohr und seinen Inhalt angelegt wird, wobei die induzierte Spannung auf das Vorhandensein oder Fehlen von Schlacke in dem Inhalt schließen lässt; **gekennzeichnet durch**
Befestigen des Gabelspulenhalters (10) an einem Rohrmanipulator (30), der so eingerichtet ist, dass er das Rohr manipuliert; und
**dadurch** die Spulen (20, 24) feststehend in Bezug auf das Rohr hält.

2. Verfahren nach Anspruch 1, das weiterhin folgende Schritte aufweist:
Bereitstellen der mindestens einen Sendespule als Toroidspule und Anordnen der mindestens einen Sendespule so, dass sie das Rohr umgibt; und
Bereitstellen der mindestens einen Empfangsspule als Toroidspule und Anordnen der mindestens einen Empfangsspule so, dass sie das Rohr umgibt.

3. Verfahren nach einem der Ansprüche 1-2, das weiterhin folgende Schritte aufweist:
Erkennen einer eventuellen turbulenten Strömung in dem Rohr; und
Ändern der Frequenz des von der mindestens einen Sendespule erzeugten elektromagnetischen Felds in dem Fall, dass eine turbulente Strömung erkannt worden ist.

4. Verfahren nach einem der Ansprüche 1 - 3, das weiterhin das Erzeugen, mittels der mindestens einen Sendespule, eines elektromagnetischen Felds mit wechselnden Frequenzen oder mehrerer elektromagnetischer Felder mit verschiedenen Frequenzen aufweist.

5. Verfahren nach einem der Ansprüche 1 - 4, **dadurch gekennzeichnet, dass** eine induzierte Spannung, die einen Wert außerhalb eines definierten Spannungsbereiches hat, auf das Vorhandensein von Schlacke schließen lässt, wobei das Verfahren weiterhin folgende Schritte aufweist:
Ermitteln des Durchsatzes des schmelzflüssigen Metalls, das durch das Rohr fließt; und
Definieren des Spannungsbereiches in Abhängigkeit von der Größe des ermittelten Durchsatzes des schmelzflüssigen Metalls.

6. Verfahren nach Anspruch 5, das weiterhin Folgendes aufweist:
Definieren eines größeren Spannungsbereiches, wenn ermittelt wird, dass die Größe des Durchsatzes des schmelzflüssigen Metalls abgenommen hat.

7. Verfahren nach einem der Ansprüche 5 - 6, **dadurch gekennzeichnet, dass** der Vorgang des Ermittelns des Durchsatzes des durch das Rohr fließenden schmelzflüssigen Metalls Folgendes aufweist:
Herstellen der Rückkopplung von einem Öffnungspositionssignal eines Schiebers an der Pfanne und Berechnen des Durchsatzes des schmelzflüssigen Metalls aus den Schieberöffnungsinformationen.

8. Verfahren nach einem der Ansprüche 5 - 6, **dadurch gekennzeichnet, dass** der Vorgang des Ermittelns des Durchsatzes des durch das Rohr fließenden schmelzflüssigen Metalls Folgendes aufweist:
Messen der Geschwindigkeit der Abnahme der Masse des Pfanneninhalts und Berechnen des Durchsatzes des schmelzflüssigen Metalls aus der gemessenen Massenabnahmegeschwindigkeit.

9. Verfahren nach einem der Ansprüche 5 - 6, **dadurch gekennzeichnet, dass** der Vorgang des Ermittelns des Durchsatzes des durch das Rohr fließenden schmelzflüssigen Metalls Folgendes aufweist:
Messen der Gießgeschwindigkeit in der Gießwanne und Berechnen des Durchsatzes des schmelzflüssigen Metalls aus der gemessenen Gießgeschwindigkeit.

10. Verfahren nach einem der Ansprüche 1 - 9, das weiterhin das Kühlen der Sende- und Empfangsspulen aufweist.

11. Vorrichtung zum Erkennen des Vorhandenseins von Schlacke in einem Rohr (4) zum Führen von schmelzflüssigem Metall von einer Pfanne (2) zu einer Gießwanne (6), mit:
mindestens einer Sendespule (20) zum Erzeugen eines elektromagnetischen Felds, das an das Rohr und seinen Inhalt angelegt werden soll;
mindestens einer Empfangsspule (24) zum Empfangen des an das Rohr und seinen Inhalt angelegten elektromagnetischen Felds und zum Erzeugen einer induzierten Spannung, wobei die induzierte Spannung auf das Vorhandensein oder Fehlen von Schlacke in dem Inhalt schließen lässt;
einem Rohrmanipulator (30), der so eingerichtet ist, dass er das Rohr manipuliert;
einem Gabelspulenhalter (10) mit mindestens zwei Zweigen, wobei ein erster Zweig (14) die mindestens eine Sendespule hält und ein zweiter Zweig (16) die mindestens eine Empfangsspule hält, wobei die beiden Zweige so angeordnet werden können, dass sich das Rohr dazwischen befindet,
**dadurch gekennzeichnet, dass** der Gabelspulenhalter (10) so an dem Rohrmanipulator (30) befestigt ist, dass die mindestens eine Sendespule und die mindestens eine Empfangsspule in Bezug auf das Rohr feststehend sind.

12. Vorrichtung nach Anspruch 11, **dadurch gekennzeichnet, dass** die Spulen Toroidspulen sind, wobei der Gabelspulenhalter (10) so eingerichtet ist, dass er jede Toroidspule so hält, dass sie das Rohr umgibt.

13. Vorrichtung nach einem der Ansprüche 11 - 12, **dadurch gekennzeichnet, dass** die beiden Zweige voneinander elektrisch getrennt sind.

14. Vorrichtung nach einem der Ansprüche 11 - 13, **dadurch gekennzeichnet, dass** eine induzierte Spannung, die einen Wert außerhalb eines definierten Spannungsbereiches hat, auf das Vorhandensein von Schlacke schließen lässt, wobei die Vorrichtung weiterhin Folgendes aufweist:
Mittel zum Ermitteln des Durchsatzes des schmelzflüssigen Metalls, das durch das Rohr fließt; und
Mittel (52) zum Definieren des Spannungsbereiches in Abhängigkeit von der Größe des gemessenen Durchsatzes.

15. Vorrichtung nach Anspruch 14, **dadurch gekennzeichnet, dass** die Mittel zum Ermitteln des Durchsatzes des durch das Rohr fließenden schmelzflüssigen Metalls Folgendes umfassen:
einen Sensor zum Erfassen eines Öffnungspositionssignals eines Schiebers an der Pfanne und
einen Prozessor zum Berechnen des Durchsatzes des schmelzflüssigen Metalls aus den Schieberöffnungsinformationen.

16. Vorrichtung nach Anspruch 14, **dadurch gekennzeichnet, dass** die Mittel zum Ermitteln des Durchsatzes des durch das Rohr fließenden schmelzflüssigen Metalls Folgendes umfassen:
ein Messgerät zum Messen der Geschwindigkeit der Abnahme der Masse des Pfanneninhalts und
einen Prozessor zum Berechnen des Durchsatzes des schmelzflüssigen Metalls aus der gemessenen Massenabnahmegeschwindigkeit.

17. Vorrichtung nach Anspruch 14, **dadurch gekennzeichnet, dass** die Mittel zum Ermitteln des Durchsatzes des durch das Rohr fließenden schmelzflüssigen Metalls Folgendes umfassen:
ein Messgerät zum Messen der Gießgeschwindigkeit in der Gießwanne und
einen Prozessor zum Berechnen des Durchsatzes des schmelzflüssigen Metalls aus der gemessenen Gießgeschwindigkeit.

18. Vorrichtung nach einem der Ansprüche 11 - 17, **dadurch gekennzeichnet, dass** die Sende- und Empfangsspulen mit Richtelementen, wie etwa einem Kern, zum Richten des elektromagnetischen Felds zu und von dem Rohr versehen sind.

19. Gießanlage mit:
einer Pfanne (2), die so eingerichtet ist, dass sie schmelzflüssiges Metall enthält;
einer Gießwanne (6), die so eingerichtet ist, dass sie schmelzflüssiges Metall aus der Pfanne aufnimmt;
einem Rohr (4), das zwischen der Pfanne und der Gießwanne angeordnet ist, wobei das schmelzflüssige Metall von der Pfanne über das Rohr zu der Gießwanne fließen kann; und
einer Vorrichtung nach einem der Ansprüche 11 - 18.

## Revendications

1. Procédé pour détecter la présence de scories dans un tube protecteur (4) destiné à guider du métal fondu depuis une poche à coulée (2) vers un bassin de coulée (6), comprenant le fait de:
procurer un porte-bobine à fourche (10) ayant au moins deux branches;
monter ladite au moins une bobine de transmission (20) à une première branche (14) et ladite au moins une bobine de réception (24) à une deuxième branche (16) du porte-bobine à fourche;
placer le porte-bobine à fourche de telle façon qu'une ligne droite imaginaire tracée entre ladite au moins une bobine de transmission et ladite au moins une bobine de réception croise le tube protecteur;
générer, au moyen d'au moins une bobine de transmission (20), un champ électromagnétique qui pénètre dans le tube protecteur et son contenu;
générer une tension induite par le biais d'au moins une bobine de réception (24) qui est soumise au champ électromagnétique qui est rentré dans le tube protecteur et son contenu, où ladite tension induite indique la présence ou l'absence de scories dans ledit contenu;
**caractérisé par** le fait de
monter ledit porte-bobine à fourche (10) à un dispositif de manipulation (30) du tube protecteur adapté pour manipuler le tube protecteur; et
maintenir ainsi les bobines (20, 24) inamovibles par rapport au tube protecteur.

2. Procédé tel que revendiqué dans la revendication 1, comprenant en plus le fait de:
prévoir ladite au moins une bobine de transmission sous une forme de tore et l'agencer de façon à entourer le tube protecteur, et
prévoir ladite au moins une bobine de réception sous une forme de tore et l'agencer de façon à entourer le tube protecteur.

3. Procédé tel que revendiqué dans l'une quelconque des revendications 1-2, comprenant en plus le fait de:
détecter l'existence d'un écoulement turbulent à l'intérieur du tube protecteur; et
modifier la fréquence du champ électromagnétique généré par ladite au moins une bobine de transmission dans le cas de la détection d'un écoulement turbulent.

4. Procédé tel que revendiqué dans l'une quelconque des revendications 1-3, comprenant en plus le fait de générer, par le biais de ladite au moins une bobine de transmission:
un champ électromagnétique à fréquences alternées, ou
plusieurs champs électromagnétiques avec des fréquences différentes.

5. Procédé tel que revendiqué dans l'une quelconque des revendications 1-4, dans lequel toute tension induite ayant une valeur à l'extérieur d'une gamme de tensions définie indique la présence de scories, le procédé comprenant en plus le fait de:
déterminer le débit du métal fondu passant à travers le tube protecteur; et
définir ladite gamme de tensions en fonction de la grandeur du débit déterminé du métal fondu.

6. Procédé tel que revendiqué dans la revendication 5, comprenant en plus le fait de:
définir une gamme de tensions plus grande si l'on détermine que la grandeur du débit de métal fondu a baissé.

7. Procédé tel que revendiqué dans l'une quelconque des revendications 5-6, dans lequel l'action consistant à déterminer le débit de métal fondu passant à travers le tube protecteur comprend le fait de:
procurer une rétroaction à partir d'un signal de position d'ouverture d'une porte coulissante au niveau de la poche de coulée et calculer le débit de métal fondu d'après l'information d'ouverture de la porte coulissante.

8. Procédé tel que revendiqué dans l'une quelconque des revendications 5-6, dans lequel l'action consistant à déterminer le débit de métal fondu passant à travers le tube protecteur comprend le fait de:
mesurer le taux de diminution en poids du contenu de la poche de coulée et calculer le débit de métal fondu à partir dudit taux de diminution en poids mesuré.

9. Procédé tel que revendiqué dans l'une quelconque des revendications 5-6, dans lequel l'action consistant à déterminer le débit de métal fondu passant à travers le tube protecteur comprend le fait de:
mesurer le taux de coulée dans le bassin de coulée et calculer le débit de métal fondu à partir dudit taux de coulée mesuré.

10. Procédé tel que revendiqué dans l'une quelconque des revendications 1-9, comprenant en plus le fait de:
refroidir lesdites bobines de transmission et de réception.

11. Dispositif pour détecter la présence de scories dans tube protecteur (4) destiné à guider un métal fondu depuis une poche de coulée (2) vers un bassin à coulée (6), comprenant:
au moins une bobine de transmission (20) destinée à générer un champ électromagnétique devant être introduit dans le tube protecteur et son contenu;
au moins une bobine de réception (24) destinée à recevoir le champ électromagnétique qui est introduit dans le tube protecteur et son contenu, et destinée à générer une tension induite, où ladite tension induite indique la présence ou l'absence de scories dans ledit contenu;
un dispositif de manipulation (30) du tube protecteur adapté pour manipuler le tube protecteur;
un porte-bobine à fourche (10) ayant au moins deux branches, une première branche (14) portant ladite au moins une bobine de transmission et une deuxième branche (16) portant ladite au moins une bobine de réception, les deux branches pouvant être placées de telle sorte que le tube protecteur se trouve entre elles; **caractérisé en ce que**
le porte-bobine à fourche (10) est monté au dispositif de manipulation (30) du tube protecteur de sorte que ladite au moins une bobine de transmission et ladite au moins une bobine de réception se trouvent dans une position stationnaire par rapport tube protecteur.

12. Dispositif tel que revendiqué dans la revendication 11, dans lequel lesdites bobines sont sous forme de tores, où le porte-bobine à fourche (10) est adapté pour maintenir chaque tore de telle manière qu'il entoure le tube protecteur.

13. Dispositif tel que revendiqué dans l'une quelconque des revendications 11-12, dans lequel lesdites deux branches sont électriquement isolées l'une de l'autre.

14. Dispositif tel que revendiqué dans l'une quelconque des revendications 11-13, dans lequel toute tension induite ayant une valeur à l'extérieur d'une gamme de tensions définie indique la présence de scories, le dispositif comprenant en plus:
un moyen destiné à déterminer le débit du métal fondu passant à travers le tube protecteur; et
un moyen (52) destiné à définir ladite gamme de tensions en fonction de la grandeur du débit mesuré.

15. Dispositif tel que revendiqué dans la revendication 14, dans lequel ledit moyen destiné à déterminer le débit du métal fondu passant à travers le tube protecteur comprend:
un capteur destiné à détecter un signal de position d'ouverture d'une porte coulissante au niveau de la poche de coulée, et
un processeur destiné à calculer le débit de métal fondu d'après l'information d'ouverture de la porte coulissante.

16. Dispositif tel que revendiqué dans la revendication 14, dans lequel ledit moyen destiné à déterminer le débit du métal fondu passant à travers le tube protecteur comprend:
un dispositif de mesure destiné à mesurer le taux de diminution en poids du contenu de la poche de coulée, et
un processeur destiné à calculer le débit du métal fondu d'après ledit taux mesuré de diminution en poids.

17. Dispositif tel que revendiqué dans la revendication 14, dans lequel ledit moyen destiné à déterminer le débit du métal fondu passant à travers le tube protecteur comprend:
un dispositif de mesure destiné à mesurer le taux de coulée dans le bassin de coulée, et
un processeur destiné à calculer le débit de métal fondu d'après ledit taux de coulée mesuré.

18. Dispositif tel que revendiqué dans l'une quelconque des revendications 11-17, dans lequel les bobines de transmission et de réception sont pourvues d'éléments directionnels, tels qu'un noyau, destinés à diriger le champ électromagnétique vers et depuis le tube protecteur.

19. Installation de coulée, comprenant:
une poche de coulée (2) adaptée pour contenir un métal fondu;
un bassin de coulée (6) adapté pour recevoir du métal fondu depuis la poche de coulée;
un tube protecteur (4) agencé entre la poche de coulée et le bassin de coulée, où le métal fondu est habilité à passer de la poche de coulée, à travers le tube protecteur, et au bassin de coulée; et
un dispositif tel que revendiqué dans l'une quelconque des revendications 11-18.
